# EUROPEAN PATENT APPLICATION

(11) **EP 2 385 068 A1**
(43) Date of publication of application: **09.11.2011**
(21) Application number: 10161806.4
(22) Date of filing: 04.05.2010
(51) Int. Cl.: C07K 16/00, C07K 16/30, C12N 15/63, A61K 39/395

(54) **Polyclonal antiserum for use in immunotherapy**

(71) Applicant: Universitätsklinikum Hamburg-Eppendorf, 20246 Hamburg (DE)
(72) Inventor: Fehse, Boris, 22527 Hamburg (DE); Ayuk Ayuketang, Francis, 22767 Hamburg (DE); Kröger, Nicolaus Martin, 22397 Hamburg (DE)
(74) Representative: UEXKÜLL & STOLBERG

(57) **Abstract**

The present invention generally relates to the field of antibody-based therapeutics and more particularly to methods for the preparation of a polyclonal antiserum in a non-human animal. The invention further relates to a polyclonal antiserum which is obtainable by the method of the invention and to the use of such antiserum as a medicament, in particular a medicament that is useful in immunotherapy, and in particular in the treatment of cancer.

## Description

The present invention generally relates to the field of antibody-based therapeutics and more particularly to methods for the preparation of a polyclonal antiserum and/or a polyclonal antibody in a non-human animal. The invention further relates to a polyclonal antiserum and a polyclonal antibody obtainable by the methods of the invention. The invention also provides a polyclonal antiserum and/or a polyclonal antibody for use as a medicament, in particular for use in immunotherapy, for example, in the treatment of cancer.

### BACKGROUND OF THE INVENTION

Polyclonal antisera are normally prepared by immunization of suitable animals, such as rabbits, with antigens of interest. The antigens are usually administered as purified polypeptides or polypeptide fragments. Polyclonal antisera are broadly used for *in vitro* analysis, although their clinical potential has been discussed and demonstrated in recent years.

Probably the most frequently used therapeutic product derived from a polyclonal antiserum is anti-thymocyte globulin (ATG). Anti-thymocyte globulin is obtained by immunization of rabbits or horses with human lymphocytic cells, e.g. with the human CD4-cell line Jurkat. ATG is used in transplantation medicine for immune suppression to prevent acute rejection of transplanted organs for prevention and treatment of graft-versus-host disease and for treating aplastic anemia. It has been speculated that polyclonal antisera may have great potential in cancer therapy. A significant advantage over the already broadly used monoclonal antibodies resides in the reduced likelihood of escape variants. Polyclonal antisera comprise a mixture of antibodies which are directed to different epitopes (or even antigens) which renders the development of escape variants less likely. On the other hand, polyclonal antisera obtained by immunization with human cell lines or human primary cells (e.g., the above mentioned ATG) are directed against a wide range of antigens, some of which are ubiquitously expressed also on normal cells. Therefore, polyclonal antisera are usually associated with undesirable side effects which limit their therapeutical use.

Accordingly, it is an objective of the invention to provide antibody-based therapeutics which minimize the risk of escape variants, and at the same time exhibit sufficient selectivity and specificity for one or more selected human and/or non-human antigens. This objective is achieved by the provision of a polyclonal antiserum and/or a polyclonal antibody as further defined in the attached claims and discussed in more detail below.

It has now been found that highly effective polyclonal antisera and antibodies for use in therapy can be obtained by immunizing non-human animals with a non-human cell that has been modified to express at least one human or non-human (e.g. viral or bacterial) antigen. The antisera and antibodies obtained in this manner are highly specific for the antigens produced by the cell that was administered to the non-human animal, and they do not show any significant non-specific immune response against human cells or tissues or components thereof, for example, HLA antigens. Therefore, the antisera and antibodies of the invention are superior to existing antisera, e.g. ATG, and are particularly suitable for therapeutic use in human patients.

Accordingly, in one aspect, the invention provides a method for the preparation of a polyclonal antiserum in a non-human animal, comprising
(a) administering to a non-human animal a non-human cell which is modified to produce one or more human and/or non-human antigens;
(b) obtaining the polyclonal antiserum from said non-human animal.

The method of the invention comprises the administration of cells to a non-human animal so as to induce an immunological response in said animal. The non-human animal to be immunized with the cells can be of any species which is known in the art to be suitable for producing antibodies against antigenic structures which are administered to said animal. Preferably, the non-human animal is a vertebrate, and more preferably a mammal, such as a non-human mammal. The mammal is preferably selected from the group consisting of rat, mouse, hamster, pig, rabbit, horse, donkey, goat, sheep, guinea pig, Ilama, and non-human primate (e.g. chimp). In a particularly preferred aspect, the non-human animal is a rodent, such as a rabbit, mouse or rat. Mice which have been found particularly suitable for use in immunization methods are, for example, Balb/c or CBA/J mice obtainable from Charles River Laboratories, Sulzfeld, Germany. The non-human animal to which the non-human cells are administered can be a transgenic animal, i.e. an animal which includes one or more genes not normally present in the genome of said animal, or one more genes which are present, but not normally expressed in the genome. For example, the non-human animal to be immunized can be a transgenic and/or transchromosomal animal, which has been modified by recombinant means to produce antibodies, e.g. humanized antibodies.

The composition comprising the non-human cell which produces the one or more human and/or non-human antigens (referred to as the "immunogenic composition" hereinafter) will be prepared in consideration of the particular mode of administration. The immunogenic composition can be administered to the non-human animal by any route which is suitable to provide for an immune response in the animal. For example, the composition can be administered via the parental, oral, mucosal, nasal, or pulmonary route. Preferably, the compositions are administered parenterally, e.g. by injection or infusion. For example, the immunological composition can be formulated for being administered intradermally, subcutaneously, intraperitoneally, intravenously, intraarterially, or intramuscularly. If it is desired to inject or infuse the antigen-containing immunogenic composition into the animal, the cells will usually be present in a solution or suspension, i.e. dissolved or suspended in a suitable liquid carrier. Liquid carriers for dissolving or suspending cells for immunization purposes are well known in the art. These carriers will normally not induce any antibodies that may be harmful for the non-human animal. Liquid carriers that may be used for the methods of the invention include, for example, physiological saline, aqueous buffers, Ringer's Solutions, and the like. The carrier may also be a solvent, such as ethanol, glycerol, propylene glycol, polyethylene glycol, and the like. Mixtures of these components may also be used as a carrier.

The immunogenic composition will be administered to the non-human animal in an overall volume typical for immunization approaches in veterinary medicine. Preferably, the volume of the composition to be administered will be less than 1 ml, preferably less than 0.8 ml, less than 0.7 ml, less than 0.6 ml, less than 0.5 ml, less than 0.4 ml, less than 0.3 ml, less than 0.2 ml, and more preferably less than 0.1 ml.

The immunogenic composition comprising the non-human cell which is used for stimulating the desired immune response in the non-human animal can also include other additives, for example, one or more adjuvants. As used herein, adjuvants are generally compounds which are capable of increasing or enhancing the pharmaceutical effects of a medicament which is co-administered to a subject. Likewise, immunological adjuvants are compounds having the capability to increase or enhance an immune response that is elicited in a subject or animal to which a particular antigen has been administered. Numerous compounds are known in the art to be effective immunological adjuvants. For example, immunological adjuvants for use in the present invention may include mineral salts, such as aluminium and calcium salts; oil-in-water-emulsions, such as SAF, AS02 or AS03; oligonucleotides with CpG motifs; or saponins, such as QS21 or QuilA. Other suitable adjuvants to be used are complete Freund adjuvant (CFA) and incomplete Freund adjuvant (IFA).

In addition, the immunogenic composition of the invention can include stabilisers, anti-oxidants, pH buffering substances, emulsifying agents, preservatives and the like, as long as these compounds do not interfere with the desired immunological activity of the non-human cell used for immunization. Pharmaceutically acceptable carriers and additives for use in the present invention are generally described in the literature, for example, in "Remington's Pharmaceutical Sciences", Mack Pub. Co., New Jersey (1991).

The number of cells to be administered to the non-human animal for antibody production will depend on the specific cell or cell line which is selected for immunization. In particular, the number of cells to be included into the immunogenic composition will depend on the amount of antigen that is presented to the immune system of the animal. For example, where the antigen is a heterologously expressed polypeptide, the expression level of said antigen in the non-human cell will be a significant parameter which influences the final cell number in the immunogenic composition. If the expression level of the antigen is low, more cells will be administered to the animal to ensure that the antigen is properly presented to the immune system of the animal. Conversely, if the antigen is strongly expressed by the non-human cell, the number of cells to be included into the immunogenic composition can be lower. Typically, the cell number for use in the immunogenic composition of the invention will be in the range of about 10⁵-10¹¹ cells per kg body weight of the animal to be immunized, preferably about 10⁷-10⁹ per kg body weight, and more preferably about 10⁸ cells per kg body weight.

The administration schedule applied can be a single dose schedule or a multiple dose schedule. For example, the immunization can comprise a primary dose administration which is followed by at least one booster immunization. Preferably, the administration schedule includes a primary dose administration and two booster immunizations which are administered within 28 days from the primary dose, e.g. after 7 days and 14 days, after 7 days and 21 days, after 7 days and 28 days, after 14 days and 21 days, after 14 days and 28 days, and the like.

At defined time points after administration of the antigen-expressing cells, blood samples are taken from the immunized animal, and the blood is processed so as to obtain the antiserum, i.e. the antibody-containing liquid component of the blood which is substantially free of clotting factors and blood cells, such as erythrocytes, lymphocytes, and platelets. Methods for the processing of blood for antiserum recovery are well known in the art. For example, the antiserum comprising the polyclonal antibodies may be obtained by centrifugation of coagulated blood, e.g. at 1000-2000 x g for 20 minutes, in order to remove the blood cells and clotting factors. The antibody-containing supernatants obtained after removal of clotting factors and blood cells may be directly used for administration to a patient. Alternatively, the so obtained antiserum may be subjected to further processing steps to fractionate it according to common methods prior to its use. For example, the polyclonal antiserum of the invention can be processed by enriching or purifying the immunoglobulin fraction. Preferably, such enriched or purified immunoglobulin fraction will comprise less than 20% (by weight) contaminating proteins (i.e. proteins other than immunoglobulins), more preferably less than 10%, and even more preferably less than 5%.

Alternatively, the polyclonal antibodies contained in the antisera of the invention may be obtained. As used herein, the term "polyclonal antibody" defines a group of antibodies which originate from different B lymphocytes and are directed to the same antigen molecule. A polyclonal antibody normally comprises antibodies which are directed to different epitopes of the antigen, but it may also comprise antibodies which are directed to the same epitope of the antigen, but which originate from different B lymphocytes. If more than one antigen has been used for immunizing the animal, the antiserum obtained from said animal will normally contain more than one polyclonal antibody. An antiserum produced by the methods of the invention, which includes one or more polyclonal antibodies, is referred to herein as a polyclonal antiserum.

Thus, in a further aspect, the invention relates to a method for the preparation of a polyclonal antibody, comprising
(a) preparing a polyclonal antiserum according to the method described above; and
(b) obtaining the polyclonal antibody from the antiserum.

Methods for separating a polyclonal antibody from an antiserum are also generally known in the art. These methods include, for example, ammonium sulphate precipitation, chromatography (gel filtration, ion exchange, DEAE-Cellulose, affinity chromatography) and any other method known in the art. For example, the polyclonal antibody may be separated from other components of the antiserum (e.g. serum proteins) by passing the antiserum several times through a chromatography column having the antigen molecule immobilized thereon so that antibodies having binding affinity for the antigen will bind to the column, whereas other serum components will be removed from the column in the flow-through. The polyclonal antibody may be enriched or substantially purified from the serum. Preferably, such substantially purified polyclonal antibody will comprise less than 20% (by weight) contaminating proteins (i.e. proteins other than immunoglobulins or other immunoglobulins having no binding affinity for the respective antigen), more preferably less than 10%, and even more preferably less than 5%. Examples of production and purification of various polyclonal antibodies are summarized by Newcombe and Newcombe (2007), Journal of Chromatography B, 848, 2-7.

The non-human cells which are used for immunization of the non-human animal have been modified to produce at least one human or non-human antigen, preferably by heterologous expression. Accordingly, the antisera resulting from the immunization will comprise, apart from antibodies directed to other cellular components of the administered cells (if any), antibodies which are directed to epitopes of the human or non-human antigen(s). If cells originating from the same species as the non-human animal to be immunized (allogeneic cells) are used for immunization, no generation of antibodies against any cellular components other than the antigen(s) of interest is to be expected. Accordingly, when used in therapy, a polyclonal antiserum or antibody of the invention will be substantially free of harmful side effects which are regularly observed when using known polyclonal antisera in clinical settings.

The non-human cell that is administered to the non-human animal can be a naturally occurring cell which has been isolated, e.g. from a tissue source by a tissue biopsy, and subsequently modified by recombinant means to produce one or more human and/or non-human (e.g. viral or bacterial) antigens. Alternatively, the cell can be a cell line, such as an immortalized cell line. The non-human cell can be derived from various non-human organisms. If the antigen to be produced is, for example, a polypeptide, any cell can be used which has been proven useful for the heterologous expression of human polypeptides. Suitable non-human cells for use in the methods of the present invention include avian, fish, arthropods, mammalian, bacterial, and yeast cells. Numerous of these cells or cell lines can be obtained from known depositories or collections, such as the American Type Culture Collection ("ATCC"), the European Collection of Cell Cultures (ECACC), or from other depositories or collections. Alternatively, they can be isolated from commonly known sources using methods well known in the art.

The cell to be administered to the non-human animal has been modified, e.g. by recombinant means, to produce one or more human and/or non-human antigens of interest. For example, a non-human cell can be generated which produces a single human or non-human (such as viral or bacterial) antigen molecule, e.g. a polypeptide antigen. Alternatively, a cell can be prepared which simultaneously produces 2, 3, 4, 5, 6, 7, 8, 9, or 10 different human or non-human antigens.

The term "human antigen" refers to any compound synthesized by human cells or tissues that is capable of eliciting an immune response upon administration into a non-human animal, such as a mouse or rabbit. As used herein, the term comprises both antigens which are found, for example, in human cells or on the surface of human cells or in body fluids under normal conditions (e.g. clusters of differentiation antigens) and antigens which are found only under pathological conditions (e.g. tumor-associated antigens). Human polypeptides, which are encoded by the human genome, are the most preferred human antigens according to the invention. Preferably, the human antigen(s) selected for expression according to the invention are human pathology-related antigens, i.e. antigens which are only detected in human cells or tissues in a disease state, or which are detected in significantly increased or decreased amounts in said disease state.

According to the invention, the one or more antigens to be expressed by the non-human cell may also include one or more non-human antigens, in particular viral antigens. As used herein, a viral antigen includes a complete virus or a fragment of a virus, a virus-like particle, a polypeptide which is encoded and expressed by the virus or any other portion of the virus that stimulates an immunological response upon administration in a non-human animal, such as a mouse or rabbit. The present invention can be used for producing polyclonal antisera or antibodies directed to a variety of different viral antigens. The source of the viral antigen is not limited to certain types of viruses. Instead, antigens from any pathogenic virus may be used. For example, viral antigens for use in the present methods comprise, but are not limited to, antigens derived from coronaviridae, herpesviridae (e.g. antigens from herpes simplex virus (HSV) types 1 and 2, varicella zoster virus (VZV), Epstein-Barr virus (EBV), cytomegalovirus (CMV) and the like), reoviridae, picornaviridae (e.g. antigens from polioviruses), togaviridae (e.g. antigens from rubella virus, dengue virus, etc.), flaviviridae (e.g. antigens from hepatitis A virus (HAV), hepatitis B virus (HBV), hepatitis C virus (HCV), the delta hepatitis virus (HDV), hepatitis E virus (REV) and hepatitis G virus (HGV)), rhabodoviridae (e.g. antigens from rabies virus), paramyxoviridae (e.g. antigens from mumps virus, measles virus, respiratory syncytial virus, etc.), orthomyxoviridae (e.g. antigens from influenza virus types A, B, C), arenaviridae, and the like. The antigens may also be derived from retroviruses, such as HIV-1 or HIV-2, simian immunodeficiency virus (SIV), and the like.

The skilled person will readily be able to select suitable antigens from one or more of the above viruses based on the information available in the state of art. Preferably, the antigens selected for the generation of polyclonal antisera or antibodies are antigens from the virus envelope. For example, where the selected viral antigens are derived from HIV, the antigens may correspond to the gp41, gp120, gp160, or gp140 envelope protein or to an immunologically active fragment thereof. Where the selected antigen stems from influenza virus, one may use the envelope glycoproteins HA and NA of influenza A for generating the immune response. Antigens from herpes simplex virus (HSV) types 1 and 2 suitable for use in the methods described herein include, for example, HSV-1 and HSV-2 glycoproteins gB, gD and gH, and glycoproteins CMV gB and gH of cytomegalovirus.

In a particularly preferred aspect, the polyclonal antiserum and/or the polyclonal antibody of the present invention is directed to one or more HIV antigens. A pharmaceutical composition containing such polyclonal antiserum and/or the polyclonal antibody is useful for treating or diagnosing HIV infections, e.g. in a post-exposure treatment regimen.

Similarly, the one or more antigens to be expressed by the non-human cell may also include one or more bacterial antigens, e.g. polypeptides expressed by bacteria. In a preferred aspect, the antigen may be derived from bacteria of the genus Neisseria, such as N. gonorrhoeae or N. meningitidis (e.g. a saccharide antigen from N. meningitidis serogroup A, B, C, W135 and/or Y); Streptococcus, such as S. pneumoniae, S. faecium, S. faecalis or S. pyogenes; Haemophilus, such as H. influenzae (e.g. H. influenzae B), H. ducreyi, H. aegyptius, H. parainfluenzae, H. vaginalis, H. haemolyticus, H. parasuis, or H. somnus; Staphylococcus, such as S. aureus, S. epidermidis, S. haemolyticus, S. lugdunensis; Bacillus, such as B. anthracis; Yersinia, such as Yersinia, such as Y. pestis, Y. pseudotuberculosis or Y. enterocolitica; Vibrio, such V. cholerae; Bordetella, such as B. pertussis, B. parapertussis; B. bronchioseptica; Chlamydia, such as C. trachomatis or C. pneumoniae; Treponema, such as T. pallidum or T. pertenue; Clostridium, such as C. perfringens, C. tetani, C. septicum or C. gigas; Mycobacterium, such as M. tuberculosis or M. leprae; Listeria, such as L. monocytogenes; Salmonella, such as S. typhi, S. paratyphi, S. enteritidis, or S. typhymurium; Salmonella, such as S. enteritidis; Shigella, such as S. dysenteriae, S. flexneri or S. sonnei; Serratia, such as S. marcescens; Moraxella, such as M. catarrhalis and Helicobacter, such as H. pylori. The antigens are preferably polypeptides or saccharides. Where a saccharide antigen is used, it is preferably conjugated to a carrier protein to enhance immunogenicity. Preferred carrier proteins are, for example, bacterial toxins or toxoids. Other suitable carrier proteins are well known in the art.

The one or more human and/or non-human antigens are preferably peptides or polypeptides which are produced by the non-human cell via heterologous expression. The cell can furthermore be modified to produce one or more non-peptidic human and/or non-human antigens, for example, antigenic polysaccharides, such as bacterial capsular polysaccharides. The cells used for immunization may also be modified to produce different types of human and/or non-human antigens, such as one or more polypeptides and one or more polysaccharides.

It is preferred that the one or more antigens are peptides or polypeptides, preferably surface peptides or polypeptides, which are derived from human cells or tissues or from a microorganism, such as viruses, bacteria, yeasts, and the like, which are heterologously and/or ectopically expressed in the modified non-human cell. For example, the molecule to be used in the immunogenic composition for producing antibodies can be a naturally occurring human polypeptide of full length, or a fragment or variant of such polypeptide. If fragments of an antigenic polypeptide are used, it is preferred to analyze these fragments beforehand to confirm that they include an amino acid sequence which is a suitable linear or conformational epitope. Methods for identifying linear and conformational epitopes based on a given amino acid sequence are well known in the art. In a particular preferred embodiment of the invention, which is discussed in more detail below, the one or more antigens which are produced in the cells are antigenic polypeptides which are known to be involved in cancer.

In a first step, the human and/or non-human antigenic polypeptides for which expression is desired are selected depending upon the intended use of the polyclonal antiserum or antibody. For example, if the antiserum or antibody is for use in treating specific types of cancer, the polypeptides selected for expression in the non-human animal typically represent cancer-specific molecules (which occur only in or on the surface of cancer cell) or cancer-associated molecules (which occur in increased or decreased amounts in or on the surface of cancer cells compared to healthy cells), e.g. tumor marker molecules. Polynucleotides encoding these polypeptides (or epitopecontaining fragments thereof) are subsequently generated and cloned into an expression construct suitable for being transferred into the host cell. The expression in the host cell can be constitutive or regulated (e.g. inducible). Preferably, the antigenic polypeptides are membrane-bound polypeptides, i.e. they are presented on the cell surface of the cell after expression.

In a preferred aspect, the methods of the invention involve the expression of one or more human polypeptides in a mammalian expression system, i.e. the non-human cell selected for expression is a mammalian cell, e.g. from a mouse, rat, hamster, or non-human primate. Non-human mammalian cells which have been proven useful for the heterologous expression of human polypeptides include primary cells and tissues (e.g. murine bone marrow cells, haematopoietic stem cells, murine lymphocytes, muscle tissue of different species, hepatocytes, and the like) as well as cultured cells (e.g. Chinese hamster ovary (CHO) cells, monkey kidney cells (COS), baby hamster kidney (BHK) cells, immortalized murine blood lymphocytes (see examples below), and the like).

Mammalian expression systems and appropriate expression constructs are widely known in the art. The expression construct can be, for example, a viral or non-viral expression vector which is useful for introducing the polynucleotides into a cell for subsequent expression of polypeptides encoded by said polynucleotides. The expression vector can be an episomal vector, i.e. one that is capable of self-replicating autonomously within the host cell, or an integrating vector, i.e. one which stably incorporates into the genome of the cell.

An expression vector will normally comprise a promoter which is functionally linked to the polynucleotide which encodes the human and/or non-human antigen(s). Suitable promoters include, but are not limited to, the cytomegalovirus promoter (CMV), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector also optionally comprises transcription termination sequences and polyadenylation sequences for improved expression of the human and/or non-human antigen(s). Suitable transcription terminator and polyadenylation signals can, for example, be derived from SV40 (Sambrook et al (1989), Molecular Cloning: A Laboratory Manual). Any other element which is known in the art to support efficient expression may be added to the expression vector, such as the Woodchuck hepatitis post-transcriptional regulatory element (wPRE).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus, adeno-associated virus (AAV), orthomyxovirus, paramyxovirus, papovavirus, picornavirus, or alphavirus. For an overview of the different viral vector systems, see Nienhuis et al., Hematology, Vol. 16: Viruses and Bone Marrow, N.S. Young (ed.), 353-414 (1993).

Retroviral vectors are particularly preferred. Retroviral vectors normally function by transducing and integrating the selected polynucleotide into the genome of the target cell. The retroviral vectors can be derived from both of the subfamilies, orthoretroviruses and spumaviruses. For example, vectors from Murine Sarcoma Virus, Bovine Leukemia, Virus Rous Sarcoma Virus, Murine Leukemia Virus, Mink-Cell Focus-Inducing Virus, Reticuloendotheliosis Virus, Avian Leukosis Virus can be used. In a particularly preferred aspect, the viral vector used for introducing the polynucleotide encoding the human and/or non-human antigen(s) is a lentiviral vector, such as a LeGO vector (e.g. LeGO iG2, SEQ ID NO:5). The skilled person will furthermore be able to combine portions derived from different retroviruses, such as LTRs, tRNA binding sites, and packaging signals to provide a recombinant retroviral vector. These retroviral vectors are then normally used for producing transduction competent retroviral vector particles. For this purpose, the vectors are introduced into suitable packaging cell lines, such as those described in US patent 5,591,624. Retrovirus vectors can also be constructed for site-specific integration into the DNA of the host cell by incorporating a chimeric integrase enzyme into the retroviral particle (see WO 96/37626).

Alternatively, non-viral vector systems may also be used for introducing the polynucleotide encoding the antigen(s) into the non-human cell. Non-viral vector systems typically involve non-viral expression vectors which are introduced into the cell, e.g. by liposome-mediated transfer, direct injection of naked DNA, or the gene gun technology.

Several mammalian expression systems are distributed by different manufacturers and can be employed in the present invention, such as plasmid- or viral vector based systems, e.g. LENTI-Smart™ (InvivoGen), GenScript^{®} Expression vectors, pAd-VAntage™ (Promega), ViraPower™ Lentiviral and Adenoviral Expression Systems (Invitrogen).

Apart from mammalian expression systems, the non-human cell which is used in the immunogenic composition of the present invention may also be of non-mammalian nature. For example, the non-human cell may be a bacterial, insect or yeast cell. Accordingly, the expression of the antigen(s) can involve commonly known expression systems which make use of such cells. For example, the human antigen of interest can be expressed in insect cells (e.g. in cells of *Aedes aegypti, Autographa californica, Bombyx mori, Spodoptera frugiperda* und *Trichoplusia ni*, etc.) by use of a baculovirus expression system such as the Bac-to-Bac Baculovirus expression system (Invitrogen, San Diego, California, USA). Alternatively, expression can be performed in bacterial cells, for example, cells of *Bacillus subtilis*, and *Escherichia coli.* For example, the QIAgenes Expression Kits for *E. coli* of Qiagen (Hilden, Germany) can be used. If expression in yeasts is desired, several expression vectors have been developed. Amongst others, vectors are available for *Candida albicans, Hansenula polymorpha, Kluyveromyces fragilis, Pichia pastoris, Saccharomyces cerevisiae* and *Schizosaccharomyces pombe.* Also, commercial expression systems based on yeasts are available, for example, the EasySelect™ Pichia Expression Kit (Invitrogen, San Diego, California, USA) may be used.

In a preferred embodiment, the cell used for immunization purposes is derived from an animal which is from the same or a related species as the non-human animal which receives said cell in the immunization approach. In this way, an undesired xenogenic immune response is substantially prevented. For example, if the cell selected for the production (e.g. expression) of the human and/or non-human antigen(s) is a murine cell, the animal used for immunization may be any rodent, such as a rat, hamster, guinea pig, and the like. More preferably, the cell used for immunization is allogenic with respect to the animal, which means that the cell is derived from an animal which is genetically different, but belongs to the same species as the animal used for vaccination. Stated differently, an allogenic cell refers to a cell that is not derived from the individual animal to which the allogenic cell is administered for immunization. For example, if the non-human animal used for the production of the antisera is a mouse, then an allogenic cell suitable for immunization would be a murine cell derived from another mouse strain, such as from an immortalized murine cell line. Thus, in one aspect, the cell modified to produce (e.g. express) one or more human and/or non-human antigen(s) is a murine cell, and the animal vaccinated by the cell is a mouse. In a further aspect, the cell modified to produce (e.g. express) one or more human and/or non-human antigen(s) is a rabbit cell, and the animal vaccinated by the cell is a rabbit. In still a further aspect, the cell modified to produce (e.g. express) one or more human and/or non-human antigen(s) is a rat cell, and the animal vaccinated by the cell is a rat.

The antigen, which is produced (e.g. expressed) by the non-human cell, or a defined combination of 2 or more antigens (e.g. 2, 3, 4, 5, 6, 7, 8 or more), is preferably suitable to identify a cell as being a target cell involved in a specific disease. For example, the antigen or combination of antigens may be characteristic for a cancer cell, such as a cell occurring in multiple myelomas. For example, a polypeptide surface marker molecule that is known to occur in specific cancer cells may be cloned and expressed in a suitable non-human cell by common techniques. The complete polypeptide antigen can be expressed, i.e. the polypeptide at full length. Alternatively, fragments of a polypeptide can be expressed which harbour epitopes, e.g. linear or conformational epitopes, which are suitable for generating antibodies. For example, if the polyclonal antiserum to be generated shall be used in the treatment of multiple myeloma, the cell used in the immunization approach can be modified to express one or more myeloma-associated antigens, such as CD19 (SEQ ID NO:1), CD38 (SEQ ID NO:2), CD40 (SEQ ID NO:3) and CD138 (SEQ ID NO:4). Preferable, the cell used in the immunization approach expresses all of the above myeloma-associated antigens. The antigens can be expressed in a suitable non-human cell that is subsequently administered to the non-human animal for antibody production.

Variants of the selected antigenic polypeptides can also be used provided that these molecules are still capable of inducing a suitable antibody response upon administration. In particular, variants shall include polypeptides which, naturally or by recombinant means, differ from the original reference amino acid sequence by one or more amino acid substitutions, deletions or additions. For example, a variant polypeptide of the above-mentioned antigens CD19, CD38, CD40 and CD138 may have an amino acid sequence with 2, 3, 4, 5, 6, or up to 10, 20, 30 or more positions in the sequences of SEQ ID NO:1, SEQ ID NO:2, SEQ ID NO:3, or SEQ ID NO:4, respectively, which are altered by the substitution of amino acids.

The amino acid substitutions can be conservative or non-conservative. It is preferred that the substitutions are conservative substitutions, i.e. a substitution of an amino acid residue by an amino acid of similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also considered as variants are those polypeptides which differ from the original antigenic polypeptide by one or more (e.g. 2, 3, 4, 5, 10, or 15) additional amino acids. These additional amino acids may be present within the amino acid sequence of the original polypeptide (i.e. as an insertion), or they may be added to one or both termini of the polypeptide. Basically, insertions can take place at any position provided that the addition of amino acids does not impair the capability of the polypeptide to induce antibodies upon administration into a suitable non-human animal. Moreover, variants also comprise those polypeptides in which, compared to the original polypeptide, one or more amino acids are lacking. Such deletions may affect any amino acid position provided that it does not impair the immunological activity of the original polypeptide antigen, e.g. the capability of producing antibodies.

Variants of the polypeptide antigen also refer to polypeptides with structural modifications relative to the naturally occurring polypeptide antigen, such as modified amino acids. According to the invention, modified amino acids are amino acids which have been modified either by natural processes, such as processing or post-translational modifications, or by chemical modification processes known in the art. Typical amino acid modifications comprise phosphorylation, glycosylation, acetylation, acylation, branching, ADP ribosylation, crosslinking, disulfide bridge formation, formylation, hydroxylation, carboxylation, methylation, demethylation, amidation, cyclization and/or covalent or non-covalent bonding to phosphotidylinositol, flavine derivatives, lipoteichonic acids, fatty acids or lipids. Such modifications have been extensively described in the literature, e.g., in Proteins: Structure and Molecular Properties, T. Creighton, 2nd edition, W. H. Freeman and Company, New York (1993).

Normally, variants of a polypeptide antigen exhibit a significant amino acid sequence identity compared to the original polypeptide. Preferably, the amino acid identity amounts to about 60%, 65%, 70%, 75%, 80%, 85%, 90%, 95%, and more preferably to more than 96%, 97%, 98%, or 99%.

In many cases, it might not be necessary to express the full-length antigenic polypeptide. Instead, it will be sufficient to express antigenic fragments of the full-length antigenic polypeptides or their variants as defined above, i.e. fragments comprising epitopes which allow for the generation of antibodies. Thus, the present invention also comprises the use of immunologically active fragments of the human polypeptides, e.g. the polypeptides depicted in SEQ ID NO:1, SEQ ID NO:2, SEQ ID NO:3, or SEQ ID NO:4, or their variants for the production of the desired polyclonal antisera. As used herein, fragments of peptides or polypeptides of the invention are peptides or polypeptides which differ from the corresponding reference peptide or polypeptide by the lack of one or several amino acids at the N terminus and/or the C terminus of the peptide or polypeptide, wherein at least part of the immunological activity, i.e., its antigenic properties, is retained. In order to be used in the immunogenic composition of the invention, the fragment of the antigenic polypeptide has to comprise one or more epitopes, i.e. a specific antigenic amino acid sequence of at least 4, 5, 6, 8 or 10 amino acids.

It is preferred that the variants of the antigenic polypeptides or immunologically active fragments of the antigenic polypeptides or their variants will retain most of the immunological activity of the original full-length polypeptide from which they are derived. It is particularly preferred that the variants or fragments have at least about 50%, at least about 75%, preferably up to 80%, 85%, 90% or even up to 99% of the activity of the antigen polypeptide selected for immunization, e.g. the polypeptide shown in SEQ ID NO:1, SEQ ID NO:2, SEQ ID NO:3 or SEQ ID NO:4.

The selected human and/or non-human antigens may also be used in the form of fusion polypeptides. As used in the context of the present invention, the term "fusion polypeptide" generally refers to a non-naturally occurring hybrid polypeptide comprising at least two polypeptides or polypeptide fragments which do not naturally occur adjacent to each other. In particular, the selected antigenic polypeptide or an immunologically active fragment thereof can be fused to another peptide or polypeptide sequence which enhances cloning, expression, or presentation of the antigenic polypeptide of the invention by macrophages or dendritic cells of the animal which is immunized with the antigen. Various heterologous T cell epitopes have been shown in the art to increase immunogenicity of proteins to which they were fused, e.g. sequences derived from Diphteria toxin (Pillai et al. (1995), Infect. Immun. 63(4): 1535-1540).

Apart from the selected polypeptide or peptide antigen, the non-human cell selected for administration may further be modified to express other polypeptides that enhance or improve the immunological response of the immunogenic composition. For example, the cells may be modified to express immunomodulating molecules, such as immunomodulating cytokines. Suitable cytokines include interleukins, including, but are not limited to, interleukin-1 alpha, interleukin-1 beta, interleukin-2, interleukin-3, interleukin-4, interleukin-6, and interleukin 12 (IL- 12); interferons, including, but are not limited to, interferon-alpha and interferon-gamma; granulocyte colony stimulating factor (GCSF)or granulocyte-monocyte colony stimulating factor (GM-CSF).

The novel polyclonal antisera and antibodies disclosed herein are useful in different fields of medicine. In particular, these antisera and antibodies can be employed in the treatment and/or diagnosis of diseases or disorders for which therapies have been developed that make use of monoclonal antibodies. A number of therapeutically highly effective monoclonal antibodies have been already approved for use in humans or are currently the subject of clinical phase III studies. For example, monoclonal antibodies have been approved or contemplated for the treatment of autoimmune diseases, such as rheumatoid arthritis (targeting the interleukin 6 receptor, tumor necrosis factor alpha or the CD-20 antigen on B lymphocytes), Crohn's disease (targeting tumor necrosis factor alpha), Colitis ulcerosa (targeting tumor necrosis factor alpha), Bechterew's disease (targeting tumor necrosis factor alpha); dermatologic disorders, such as psoriasis (targeting the CD11a antigen); ophthalmic disease, such as macula degeneration (targeting vascular endothelial growth factor A); bronchial asthma (targeting the Fc portion of IgE); osteoporosis (targeting the RANK ligand); graft-versus-host disease (targeting the interleukin 2 receptor) and other diseases or disorders. These diseases or disorders should likewise be accessible for a treatment using the polyclonal antisera and antibodies described herein. Further, the polyclonal antisera and antibodies of the invention are suitable for the post-exposure treatment of HIV and other viral infections.

In a particular preferred aspect, the disease to be treated or diagnosed by the polyclonal antisera and antibodies of the invention is cancer, which means that the cell administered to the non-human animal produces an antigenic molecule that is involved, e.g. overexpressed, in cancer. Preferably, the antigen is specific for only a single type of cancer. According to the invention, the antigenic peptide or polypeptide may be a surface marker or a combination of surface markers which is known to be present on malignant cells, e.g. on cells of pancreatic cancer, breast cancer, colon cancer, brain cancer, prostate cancer, lung cancer, bronchial cancer, liver cancer, bladder cancer, skin cancer, ovarian cancer, cervical cancer, head and neck cancer, hematological cancers, cervical cancer, ovarian cancer, stomach cancer, kidney cancer, uterine cancer, bone cancer, esophageal cancer, laryngeal cancer, nasopharyngeal cancer, oropharyngeal cancer, testicular cancer, vulvar cancer, hepatoma, salivary gland carcinoma, thyroid cancer, parathyroid cancer, lymphomas, sarcomas, gallbladder cancer, germ cell cancer, multiple myeloma, small intestine cancer, thymus cancer, and the like. As used herein, cancer diseases also include different types of blood or bone marrow cancers, in particular leukemias such as acute lymphocytic leukemia (ALL), chronic lymphocytic leukemia (CLL), acute myelogenous leukemia (AML), chronic myelogenous leukemia (CML), hairy cell leukemia (HCL), T-cell prolymphocytic leukemia (T-PLL), and the like. According to a preferred embodiment, the cancer from which the one or more antigens are derived is chronic lymphocytic leukemia (CLL) or multiple myeloma.

Polyclonal antisera or antibodies of the invention, which have been raised against cancer antigens, can be used alone in the form of a monotherapy, or in a combination therapy together with radiation and/or other chemotherapeutic drugs. For example, the polyclonal antibodies can be administered before or after radiation of the cancerous tissue. Radiation can include electromagnetic radiation (gamma rays, x-rays) or particle beams (protons, electrons, neutrons, heavy ions or pi-mesons). The radiation dose to be applied will depend both upon the type of cancer to be treated and the type of radiation. The skilled person will have no problems to select appropriate doses for a radiation that is applied in combination with the polyclonal antisera and/or antibodies of the invention.

When used in combination therapy, the polyclonal antisera or antibodies of the invention are preferably used together with commonly known anti-proliferative agents. Chemotherapeutic agents which may be combined for use with the polyclonal antisera or antibodies according to the invention include alkylating agents, e.g. ethylenimines and methylamelamines, such as thiotepa, altretamine, triethylenemelamine, trietylenephosphoramide, triethylenethiophosphaoramide and trimethylolomelamine; alkyl sulfonates such as busulfan (Myleran®), treosulfan (Ovastat®) and piposulfan; nitrogen mustards such as ifosfamide (Holoxan®, IFO-cell®), chlorambucil (Leukeran®), melphalan (Alkeran®), estramustine, chloronaphazine, cholophosphamide, mechlorethamine, novembichin, phenesterine, prednimustine, trofosfamide; nitrosureas such as fotemustine, lomustine, carmustine, chlorozotocin, nimustine, ranimustine; aziridines such as carboquone, benzodopa, meturedopa, uredopa; purine analogs such as 6-mercaptopurine, azathioprine (Azaiprin®, AZAMEDAC®, Imurek®, Zytrim®), fludarabine, fludarabine phosphate (Fludara®), cladribine (Leustatin®), mercaptopurine (MERCAP®, Puri-Nethol®), pentostatine (Nipent®), thioguanine (Thioguanin-Wellcome®); pyrimidine analogs such as ancitabine, azacitidine, 6-azauridine, carmofur, cytarabine (Alexan®, ARA-cell®, Udicil®), dideoxyuridine, doxifluridine, enocitabine, 5-fluorouracile (Efudix®, Fluoroblastin® Ribofluor®), gemcitabine (Gemzar®), floxuridine; and nitrosureas such as carmustine (Carmubris®), chlorozotocin, fotemustine (Muphoran®), lomustine (Cecenu®, Lomeblastin®), nimustine (ACNU®), ranimustine, bendamustine (Ribomustin ®); platinum compounds, such as cisplatin (Platiblastin®, Platinex®) or carboplatin (Carboplat®, Ribocarbo®); folic acid analogs such as methotrexate (Farmitrexat®, Lantarel®, METEX®, MTX Hexal®); Anthracyclines such as doxorubicin (Adriblastin®, DOXO-cell®), daunorubicin (Daunoblastin®), epirubicin (Farmorubicin®), idarubicin (Zavedos®), Amsacrin (Amsidyl®) or mitoxantron (Novantron®); vinca alkaloids and taxanes such as vinblastin (Velbe®), vindesin (Eldisine®), vinorelbin (Navelbine®), docetaxel (Taxotere®) or paclitaxel (Taxol®); oxazaphosphorines such as cyclophosphamid (CYCLO-cell®, Cyclostin®, Endoxan®) or trofosfamid (Ixoten®), and the like. It is to be understood that variants, derivatives and salts of the above compounds may also be used in the practice of the present invention. The skilled person will readily be able to determine amounts of the above compounds which are useful when being administered in combination with a polyclonal antiserum or a polyclonal antibody of the present invention.

When used for treating cancer, it is also possible to combine the polyclonal antisera or antibodies of the invention with monoclonal antibodies which are currently in clinical use. For example, a combination with the anti-angiogenically effective monoclonal antibody Bevacizumab (Avastin®) is contemplated herein. Likewise, it will be possible to combine the antisera and/or antibodies of the invention with monoclonal antibodies which function as tyrosine kinase inhibitors, e.g. Sorafenib (Nexavar®) or Sunitinib (Sutent®). Combinations with therapeutic antibodies, such as Trastuzumab (Herceptin®), Gemtuzumab (Mylotarg®), Panitumumab (Vectibix®) or Edrecolomab (Panorex®) are also within the scope of the present invention. The skilled person will be able to select amounts of the above monoclonal antibodies which are suitable for administration in a combination with a polyclonal antiserum and /or antibody of the invention.

When used together with one of the above-mentioned anti-proliferative agents or monoclonal antibodies, the polyclonal antiserum or antibody of the invention or a composition comprising such polyclonal antiserum or antibody may be administered before, during or after administration of the anti-proliferative agents and/or monoclonal antibody. It is particularly preferred to administer the polyclonal antiserum or antibody of the invention or a composition comprising same simultaneously with any other anti-proliferative agent and/or monoclonal antibody, more preferably within the same composition. However, it is also possible to administer any anti-proliferative agent and/or monoclonal antibody that is to be used together with a polyclonal antiserum or antibody of the invention within 2, 4, 6, 8, 10, 12 or 14 days before or after the administration of said polyclonal antiserum or antibody. For example, the polyclonal antiserum or antibody may be used to initiate or terminate a chemotherapy cycle. The skilled person will be able to design suitable administration schedules for a given patient based on his general knowledge and some routine experimentation.

In a further aspect, the invention relates to the use of a polyclonal antiserum or antibody of the invention or a composition comprising the antiserum or antibody as a medicament. The polyclonal antiserum (or an antibody-containing fraction thereof) or the polyclonal antibody or a composition comprising said antiserum or antibody are preferably for use in a method of treating or diagnosing cancer. As demonstrated in the examples, a polyclonal antiserum prepared according to the method disclosed herein shows a significant cytotoxic activity against cancer cells. The observed cytotoxic effect may be due to complement-dependent cytotoxicity, direct induction of apoptosis as well as antibody-dependent cell cytotoxicity.

The therapeutic effects can be further enhanced by conjugation of the antibodies in the polyclonal serum with cytotoxic agents. Suitable cytotoxic agents for conjugation purposes include the chemotherapeutic agents mentioned above, and also radioactive substances (see, e.g., Griffiths et al. (1991), Cancer Research 51, 4594-4602) and toxins (e.g. reviewed by Pastan and Kreitman (1998), Adv Drug Deliv Rev, 31: 53-88). Likewise, diagnostic conjugates can be provided in which the polyclonal antibodies of the invention are coupled to moieties which can be detected by common means.

The polyclonal antiserum or antibody obtained by the methods described above will be formulated to a pharmaceutical or diagnostic composition according to commonly known methods. Accordingly, the present invention also relates to a pharmaceutical or diagnostic composition comprising a polyclonal antiserum or antibody of the present invention or an antibody-containing fraction of said antiserum. Apart from the polyclonal antiserum (or a fraction thereof) or the polyclonal antibody, the composition usually comprises a pharmaceutically acceptable carrier. Suitable carriers include, but are not limited to, water, saline, glycerol, Ringer's Solutions, ethanol, dextrose solution, and the like. Further examples of suitable carriers for compositions comprising the polyclonal antisera or antibodies of the invention are described in standard textbooks, for example, in "Remington's Pharmaceutical Sciences", Mack Pub. Co., New Jersey (1991). In addition to one or more of the above carrier compounds, the pharmaceutical or diagnostic composition may also contain further excipients, such as wetting agents, emulsifying agents, pH buffering agents, stabilizers, dyes and the like, as long as these compounds do not interfere with the desired pharmaceutical or diagnostic activity of the polyclonal antiserum or antibody of the invention. The compositions can be enclosed in ampoules, disposable syringes or multiple dose vials made of glass or plastic.

The pharmaceutical or diagnostic composition provided by the present invention will be formulated to be compatible with the intended route of administration. Different routes of administration are feasible for providing the polyclonal antiserum or antibody to the patient in need of treatment. Accordingly, the pharmaceutical or diagnostic composition of the present invention can be formulated for oral, rectal, nasal, pulmonary, transdermal, transcutaneous or parenteral administration (including subcutaneous, intramuscular, intravenous and intradermal administration). In a preferred embodiment, the pharmaceutical or diagnostic composition of the invention is formulated for being subcutaneously or intramuscularly injected or intravenously infused into the patient.

Pharmaceutical or diagnostic compositions suitable for injection will normally include sterile aqueous solutions or suspensions. Alternatively, the pharmaceutical or diagnostic composition can be formulated as a powder for extemporaneous preparation of injectable solutions or suspensions. They will typically comprise a pharmaceutically acceptable carrier, preferably a liquid carrier. Liquid carriers that may be used for the methods of the invention include, for example, sterile water, physiological saline, aqueous buffers, Ringer's Solutions, and the like. The carrier may also be a solvent, such as, ethanol, glycerol, propylene glycol, polyethylene glycol, and the like. Mixtures of these components may also be used as a carrier.

The pharmaceutical or diagnostic composition for injection must be sterile and should be fluid to allow administration by a syringe. The pharmaceutical or diagnostic composition can include commonly known additives which improve their effectiveness and/or allow for their storage. For example, the composition of the invention can include preservatives, such as phenol, ascorbic acid, parabens, chlorobutanol, thimerosal, and the like, to protect the composition against contaminations with bacteria and fungi.

Sterile powders for the preparation of injectable suspensions may be obtained by common methods, involving, e.g., vacuum drying and freeze-drying.

The polyclonal antiserum or antibodies are preferably administered by intravenous infusion over a period of about 10 to 300 min, preferably about 20 to 240 min, more preferably about 30 to 180 min, e.g. about 60 min, about 90 min, about 120 or about 150 min. The specific treatment protocol will be readily designed by the physician practising the invention.

The dosage level employed for administration of the antiserum or antibody into a patient, e.g. a cancer patient, will depend on several different factors. The most effective dose and maximum tolerated dose will be determined by the skilled person in standard clinical trials. It will be appreciated that the concrete amount of the polyclonal antiserum or antibody for being administered to the patient will depend on several other factors, such as age, weight and general health state of the patient, as well as the nature and severity of the medical symptoms to be treated. The amount will in any individual case be determined by one of ordinary skill in the art using routine experimentation.

In a further aspect, the invention relates to a polyclonal antiserum which is obtainable by a method as defined in the above description and the enclosed claims. The polyclonal antiserum prepared according to the method of the invention is preferably for use as a medicament, e.g. for use in immunotherapy. In a particularly preferred embodiment, the medicament is for use in cancer therapy. Accordingly, the invention also relates to the use of a polyclonal antiserum as defined elsewhere herein for the preparation of a medicament for use in immunotherapy, more preferably cancer therapy. Similarly, the invention relates to a polyclonal antibody which is obtainable by a method as defined above and also in the enclosed claims. The polyclonal antibody prepared according to the method of the invention is preferably for use as a medicament, e.g. for use in immunotherapy. In a particularly preferred embodiment, the medicament is for use in cancer therapy. The invention also relates to the use of a polyclonal antibody as defined herein for the preparation of a medicament for use in immunotherapy, more preferably cancer therapy.

The invention therefore particularly relates to a polyclonal antiserum or an immunoglobulin-containing fraction thereof as described above, to a polyclonal antibody as described above or to pharmaceutical or diagnostic composition comprising such antiserum, antiserum fraction or antibody, for use in a method of treating or diagnosing cancer.

Of course, the polyclonal antisera and antibodies obtained by the methods of the present invention may also conveniently be used *in vitro* in immunoassay, for example, in diagnostic immunoassays which are directed to the detection of cells (e.g. abnormal cells, such as cancerous cells) in a biological sample, such as a tissue or body fluid sample. The polyclonal antisera and antibodies may thereby facilitate the diagnosis of diseases such as cancer.

The term "about" as used herein in the context with a numerical range or value shall mean ±10 percent of the stated numerical range or value.

### BRIEF DESCRIPTION OF THE FIGURES

Fig. 1 shows the cytotoxic effect of the polyclonal antiserum 3395 obtained in accordance with the invention against cells of human Burkitt's lymphoma cell line Raji.
Fig. 2 shows that pre-incubation of CD40+ Raji cells with AMAG, but not with wildtype serum, resulted in the specific inhibition of binding of a monoclonal anti-CD40 antibody.

### EXAMPLES

### Example 1: Cloning of lentiviral vectors

cDNAs of different myeloma-specific antigens (CD19, CD38, CD40, CD138) were obtained from ImaGenes (Berlin, Germany) and amplified with Platinum Pfx PCR Polymerase (Invitrogen) via PCR to introduce adequate restriction sites for the subsequent cloning. The following primers were used:
CD19fw: ATAGCGGCCGCCACCATGCCACCTCCTC (SEQ ID NO:6)
CD19rv: TATTGTACATCACCTGGTGCTCCAGGT (SEQ ID NO:7)

CD38fw: ATAGCGGCCGCCACCATGGCCAACTGCGAGTTCAG (SEQ ID NO:8)
CD38rv: TATTGTACATCAGATCTCAGATGTGCAAGATGAATC (SEQ ID NO:9)

CD40fw: ATAGCGGCCGCCACCATGGTTCGTCTGCCTCTGC (SEQ ID NO:10)
CD40rv: TATTGTACATCACTGTCTCTCCTGCACTG (SEQ ID NO:11)

CD138fw: ATAGCGGCCGCCACCATGAGGCGCGCGG (SEQ ID NO:12)
CD138rv: TATTGTACATCAGGCATAGAATTCCTCCTGTTTG (SEQ ID NO:13)

PCR was performed in a final volume of 50 µl containing the following reagents:
■ 100 ng template DNA (CD19/38/40/138)
■ 5 µl 10 pmol/µl forward Primer
■ 5 µl 10 pmol/µl reverse Primer
■ 1.5 µl 10 mM dNTP's
■ 0.5 µl PFX
■ 5 µl 10x Buffer
■ 1 µl MgSO₄ 50 mM
■ H₂O ad 50 µl

Amplification was carried out using the following program:
Initial denaturation: 3 min 95°C
35 cycles (40 cycles for CD19) of
   - denaturation 30 sec 95°C
   - annealing 30 sec 55°C (61°C for CD19)
   - polymerisation (elongation) 60 sec 72°C
   - final elongation 3 min 72°C

The size of the respective PCR product was analyzed on a 1% agarose gel (CD19: about 1600 bp, CD38: about 900 bp, CD40: about 830 bp and CD138: about 930 bp). After confirming the correct size of the PCR products, said products were purified using the QIAquick PCR Purification kit (QIAGEN) in accordance with the manufacturer's instruction.

The cDNAs were then cloned into lentiviral LeGO vectors developed by the inventors (Weber et al. 2008, Molecular Therapy 16, 698-706). The following cloning procedure was used: The PCR products as well as the vector of choice (LeGO iG2; 6 µg) were incubated overnight with restriction enzymes Bsp1407I and NotI. Thereafter, reaction tubes were incubated at 65°C for 20 min. to inactivate the restriction enzymes. All mixtures were subjected to gel electrophoresis (1% agarose gel), fragments of the expected size were excised and extracted using the QIAGEN gel extraction protocol.

Taking into account the different sizes, cDNA fragments ("inserts") and vector DNA (LeGO-iG2) were incubated at ratios of approximately 1:6 in the following ligation reaction. The total DNA amount in the ligation was in the range of 700 to 1000 ng. T4 DNA ligase, buffer and ddH₂O were added according to the recommendation of the manufacturer (Fermentas). The ligation mix was incubated at 16°C for 2.5 h and thereafter used to transform competent bacteria (E. coli; DH10b) according to standard procedures. Briefly, 10 µl ligation mix were mixed with 100 µl competent bacteria and incubated on ice for 30 min. Thereafter, bacteria were subjected to a "heat shock", i.e. incubated for 45 sec at 42°C. After another short incubation on ice (2 min), the mix was transferred to 500 µl prewarmed (37°C) LB medium and incubated for 1 h at 37°C. 100 µl of this mix were disposed on an LB amp plate, the remaining part was centrifuged down and transferred to a second LB amp plate. LB amp plates were incubated o/n at 37°C, and individual bacteria clones were picked on the next day into 3-5 ml liquid LB media (also containing ampicillin) to grow bacteria for minipreps. Plasmid DNA was then isolated using QIAGEN Miniprep kits according to the instructions of the manufacturer. Integrity of cloned plasmids was confirmed using restriction analysis with suitable enzymes, particularly BSP1407I und NotI. For correct clones, maxi-prep DNA isolation was performed in accordance with the protocol of the manufacturer. Sequences were re-checked by DNA sequencing using appropriate primers. The vectors resulting from the above cloning approach were designated LeGO-CDC19, LeGO-CDC38, LeGO-CDC40, and LeGO-CDC138. The identity of the respective cDNA clones was verified by sequencing.

### Example 2: Production of lentiviral vector supernatants

Lentiviral supernatants of the above LeGO vectors were produced and titrated using HEK293T cells according to the protocol established in the laboratory of the inventors (Weber et al. (2008), Molecular Therapy 16, 698-706). Briefly, 293T packaging cells were transiently transfected with the 3^{rd} generation packaging plasmids pMDLg/pRRE and pRSV-Rev (Dull et al. (1998), J. Virol. 72:8463-8471), the vector and the VSV-G envelope plasmids (Weber et al. (2008), see above). Then, 5x10⁶ 293T cells were seeded into 10 cm Petri dishes. On the next day, all plasmids (vector, gag/pol, Rev, VSVG) were mixed at appropriate concentrations (Weber et al. 2008, see above) and H₂O was added to a final volume of 450 µl. 50 µl CaCl₂ solution was added. This DNA/CaCl₂ solution (500 µl) was added dropwise to 500 µl 2xHBS in 15 ml tubes under permanent mixing ("air bubbling"). The mixture was incubated at RT for 20 min.

For transfection, culture medium was omitted from the cells and replaced by "transfection medium" (i.e. culture medium + protmaine sulphate). Immediately thereafter, the DNA/CaCl₂ mix in HBS was added. 293T cells were incubated for 6-12 h at 37°C in an incubator (5% CO₂, humidified) before the medium was exchanged. Cell-free viral supernatants were harvested 24, 36, 48 and 60 hours after transfection and filtrated through a 0.22 µm low-protein binding sterile filter. Supernatant was aliquoted and stored at -80°C.

To determine vector titers, aliquots of the produced supernatants (containing the pseudotyped vector particles) were titrated on K562 cells. Therefore, fixed numbers of K562 cells were incubated with increasing amounts of supernatant. Gene transfer rates were analyzed at least 48 hours after transduction by fluorescence activated cell sorting (FACS) and based thereon, titers (i.e. numbers of infectious particles per ml) were calculated.

### Example 3: Preparation of antigen-expressing lymphocytes

The immortalized murine lymphocytic cell line used in the present examples was CTLL-2 (ATTC TIB-214). The cells were successively transduced with the four LeGO vectors described above. To do so, CTLL-2 cells were first incubated with LeGO-CD19-containing supernatant in the presence of protamine sulphate. After successful transduction and detection of the CD19 antigen on the cell surface via FACS analysis (Fehse et al. (2000), Molecular Therapy, 1, 448-456; Fehse et al. (2000), British Journal of Haematology 109, 644-651; Fehse et al. (2002), Gene Therapy 9, 1633-1638; Weber et al. (2008), see above 2008), the modified cells were nearly completely purified by magnetic and/or fluorescence-activated cell sorting (Fehse et al. (2000), Molecular Therapy, 1, 448-456; Fehse et al. (2000), British Journal of Haematology 109, 644-651; Fehse et al. (2002), Gene Therapy 9, 1633-1638; Weber et al. (2008), see above 2008), before transduction with the next vector (LeGO-CD38) was performed. Again, successfully transduced cells were sorted for CD38 expression. The same procedure was then repeated for LeGO-CD40 and finally for LeGO-CD138. In this manner, a cell line was prepared that was positive for all of the four antigens. This cell line designated "M-MAECl" ("Mouse-Myeloma Antigen Expressing Cells 1") was used for subsequent immunization (see below). Highly positive cells were isolated from the polyclonal cell line via fluorescence-activated cell sorting. Single clones were obtained by limiting dilution. For one of these clones it was shown that the expression of the antigens is stable for at least 10 weeks.

### Example 4: Immunization of rabbits

Each group (M-MAECl and control group) consisted of 4 rabbits. Rabbit immunization was carried out as follows: On day 0 each rabbit received 1 x 10⁸ M-MAEC1 or the same number of the unmodified murine lymphocytic cells (control group) resuspended in 3 ml of serum-free RPMI1640 Medium (Gibco) administered subcutaneously. Two intravenous boost immunizations followed on day 21 and day 28 using the same cell number and volume. Serum was collected from immunized mice on day 42.

For production of sera, whole blood was collected at final bleeding of the rabbits, allowed to coagulate and thereafter centrifuged. Serum was then collected and again centrifuged to eliminate contaminating cells. Sera were then cryopreserved until use. The serum obtained from immunization with M-MAEC1 was referred to as anti-myeloma antigen globulin (AMAG).

### Example 5: Cytotoxicity assay

Since the sera obtained in the above-described manner still contained complement components, they were inactivated by heating to 56°C for 1 hour and subsequently cryoconservated at -80°C prior to use. Active human serum was added as a source for complement to the respective experimental samples. In this way, the different volumes of sera could be used without the amount of complement being different between the distinct batches. Human Raji cells, a lymphoma cell line which is positive for the four used antigens as shown by FACS analysis, were used for the cytotoxicity assay. For each experimental sample, 1 x 10⁶ Raji cells were resuspended in 100 µl active human serum. 100 µl anti-myeloma antigen globulin (AMAG) serum obtained from example 4 above or wildtype serum, respectively, were added in concentrations decreasing from 1:2 (250 µl serum + 250 µl PBS) to 1:64 (250 µl of 1:32 serum + 250 µl PBS) and incubated for 1 hour at 37°C. Subsequently, 200 µl PBS and 10 µl propidium iodide were added to each sample and the batches were incubated in the dark for 10 minutes at 4°C. Immediately before FACS analysis, 100 µl counting beads/sample and, optionally, 4 µl 7-AAD were added. All experiments were carried out in duplicate. The incubation of Raji cells with 2 µg monoclonal antibody Rituximab (anti-CD20) served as a positive control. Cytotoxicity was analysed by means of a FACS assay. For this, the cells were stained with annexin and propidium iodide (PI). Dead cells were identified by the live gates (forward vs. side scatter) and PI staining and, optionally, also 7-AAD staining. Fixed numbers of the counting beads in every tube were measured in order to ensure that identical volumes of the cell suspensions were analyzed.

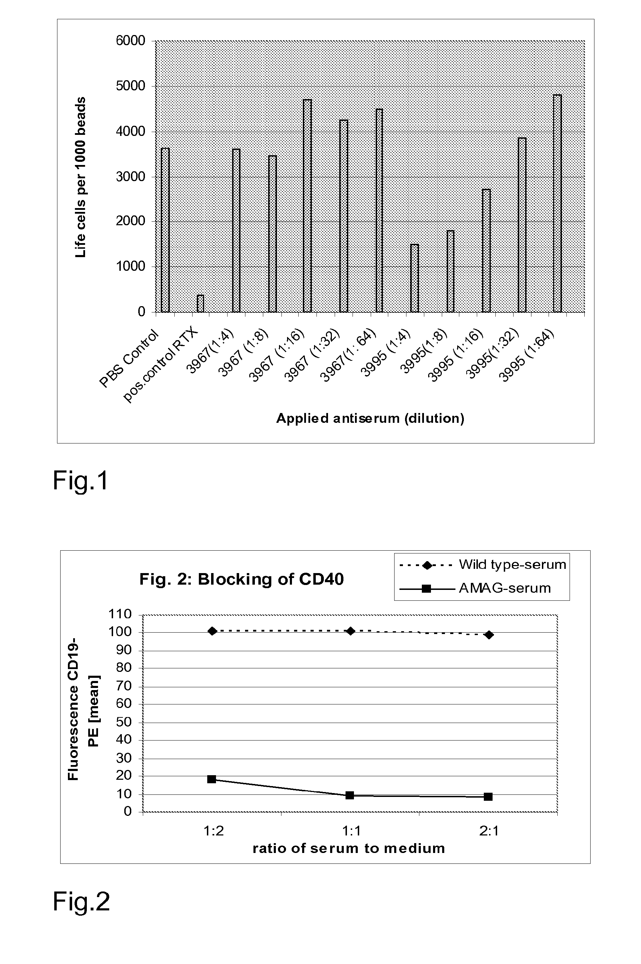

The results of the assay are shown in figure 1. As can be seen in the figure, the specific serum obtained by the method described herein exhibited a strong cytotoxic activity against Raji cells which was not observed with the control serum. The effect exerted by the monoclonal anti-CD20 (Rituximab) is shown for comparison.

### Example 6: Blocking of specific antibodies

In a further example, it was analyzed whether the immunization resulted in the generation of specific antibodies which are directed against the transduced antigens. Raji cells expressing CD40 were incubated in the concentration of 1 x 10⁷/ml (2ml total volume) with 10% AB serum for 20 minutes at room temperature. After a washing step with PBS, the cell pellet was resuspended in the same volume of PBS and aliquots of 20 µl (2x10⁵ cells) were pipetted into FACS tubes. Medium, wildtype serum or AMAG were mixed with the cells in a ratio of 2:1, 1:1 and 1:2 and incubated for 20 minutes at 4°C. Subsequently the samples were washed with PBS, stained with a monoclonal anti-CD40 PE (Phycoerythrin, a fluorescent dye) antibody and measured with a flow cytometer. The average fluorescence (CD40-PE) from 3 independent samples was determined relative to the total fluorescence of the cells that were treated with medium (100%). All used sera were inactivated for 1 hour at 56°C and subsequently cryoconservated at -80°C prior to their use. Additionally a titration of the used antibody was carried out.

The results are shown in figure 2. It can be seen that the anti-myeloma antigen globulin, but not the wildtype serum, contains specific antibodies against human CD40.

## Claims

1. Method for the preparation of a polyclonal antiserum, comprising
(a) administering to a non-human animal a non-human cell which is modified to produce one or more human and/or non-human antigens;
(b) obtaining the polyclonal antiserum from said non-human animal.

2. Method according to claim 1, wherein the non-human animal is selected from the group consisting of rat, mouse, hamster, rabbit, horse, donkey, goat, sheep, Ilama, guinea pig, and pigs.

3. Method according to any of claims 1-2, wherein the non-human cell is derived from the same species as the non-human animal.

4. Method according to any of claims 1-3, wherein the non-human cell is modified to produce more than one human and/or non-human antigen.

5. Method according to any of claims 1-4, wherein the human and/or non-human antigens are polypeptides.

6. Method according to any of claims 1-5, wherein the non-human cell is modified by transduction with a viral vector.

7. Method according to any of claims 1-6, wherein the one or more antigens are human cancer-specific or cancer-associated antigens.

8. Method according to claim 7, wherein the one or more human antigens are antigens which are characteristic for multiple myeloma.

9. Method according to claim 8, wherein the one or more antigens is selected from the group of human CD19, CD38, CD40 and CD138.

10. Method according to any of claims 1-6, wherein the one or more non-human antigens are viral or bacterial antigens.

11. Polyclonal antiserum obtainable by a method according to any of claims 1-10.

12. Method for the preparation of a polyclonal antibody, comprising
(a) preparing a polyclonal antiserum according to the method of any of claims 1-10; and
(b) obtaining the polyclonal antibody from the antiserum.

13. Polyclonal antibody obtainable by a method according to claim 12.

14. Pharmaceutical or diagnostic composition comprising the polyclonal antiserum according to claim 11 or an immunoglobulin-containing fraction thereof or the polyclonal antibody according to claim 13.

15. Polyclonal antiserum according to claim 11 or an immunoglobulin-containing fraction thereof or polyclonal antibody according to claim 13 or pharmaceutical or diagnostic composition according to claim 14, for use in a method of treating or diagnosing cancer.

16. Use of the polyclonal antiserum according to claim 11 or an immunoglobulin-containing fraction thereof, or the polyclonal antibody according to claim 13, in an *in vitro* immunoassay.


SEQUENZPROTOKOLL

<110>  Univeritätsklinikum Hamburg-Eppendorf

<120>  POLYCLONAL ANTISERUM FOR USE IN IMMUNTHERAPY

<130>  P 80744

<160>  13

<170>  PatentIn version 3.3

<210>  1
<211>  556
<212>  PRT
<213>  Homo sapiens

<400>  1


<210>  2
<211>  300
<212>  PRT
<213>  Homo sapiens

<400>  2

Met Ala Asn Cys Glu Phe Ser Pro Val Ser Gly Asp Lys Pro Cys Cys
1               5                   10                  15

Arg Leu Ser Arg Arg Ala Gln Leu Cys Leu Gly Val Ser Ile Leu Val
            20                  25                  30

Leu Ile Leu Val Val Val Leu Ala Val Val Val Pro Arg Trp Arg Gln
        35                  40                  45

Gln Trp Ser Gly Pro Gly Thr Thr Lys Arg Phe Pro Glu Thr Val Leu
    50                  55                  60

Ala Arg Cys Val Lys Tyr Thr Glu Ile His Pro Glu Met Arg His Val
65                  70                  75                  80

Asp Cys Gln Ser Val Trp Asp Ala Phe Lys Gly Ala Phe Ile Ser Lys
                85                  90                  95

His Pro Cys Asn Ile Thr Glu Glu Asp Tyr Gln Pro Leu Met Lys Leu
            100                 105                 110

Gly Thr Gln Thr Val Pro Cys Asn Lys Ile Leu Leu Trp Ser Arg Ile
        115                 120                 125

Lys Asp Leu Ala His Gln Phe Thr Gln Val Gln Arg Asp Met Phe Thr
    130                 135                 140

Leu Glu Asp Thr Leu Leu Gly Tyr Leu Ala Asp Asp Leu Thr Trp Cys
145                 150                 155                 160

Gly Glu Phe Asn Thr Ser Lys Ile Asn Tyr Gln Ser Cys Pro Asp Trp
                165                 170                 175

Arg Lys Asp Cys Ser Asn Asn Pro Val Ser Val Phe Trp Lys Thr Val
            180                 185                 190

Ser Arg Arg Phe Ala Glu Ala Ala Cys Asp Val Val His Val Met Leu
        195                 200                 205

Asn Gly Ser Arg Ser Lys Ile Phe Asp Lys Asn Ser Thr Phe Gly Ser
    210                 215                 220

Val Glu Val His Asn Leu Gln Pro Glu Lys Val Gln Thr Leu Glu Ala
225                 230                 235                 240

Trp Val Ile His Gly Gly Arg Glu Asp Ser Arg Asp Leu Cys Gln Asp
                245                 250                 255

Pro Thr Ile Lys Glu Leu Glu Ser Ile Ile Ser Lys Arg Asn Ile Gln
            260                 265                 270

Phe Ser Cys Lys Asn Ile Tyr Arg Pro Asp Lys Phe Leu Gln Cys Val
        275                 280                 285

Lys Asn Pro Glu Asp Ser Ser Cys Thr Ser Glu Ile
    290                 295                 300


<210>  3
<211>  277
<212>  PRT
<213>  Homo sapiens

<400>  3

Met Val Arg Leu Pro Leu Gln Cys Val Leu Trp Gly Cys Leu Leu Thr
1               5                   10                  15

Ala Val His Pro Glu Pro Pro Thr Ala Cys Arg Glu Lys Gln Tyr Leu
            20                  25                  30

Ile Asn Ser Gln Cys Cys Ser Leu Cys Gln Pro Gly Gln Lys Leu Val
        35                  40                  45

Ser Asp Cys Thr Glu Phe Thr Glu Thr Glu Cys Leu Pro Cys Gly Glu
    50                  55                  60

Ser Glu Phe Leu Asp Thr Trp Asn Arg Glu Thr His Cys His Gln His
65                  70                  75                  80

Lys Tyr Cys Asp Pro Asn Leu Gly Leu Arg Val Gln Gln Lys Gly Thr
                85                  90                  95

Ser Glu Thr Asp Thr Ile Cys Thr Cys Glu Glu Gly Trp His Cys Thr
            100                 105                 110

Ser Glu Ala Cys Glu Ser Cys Val Leu His Arg Ser Cys Ser Pro Gly
        115                 120                 125

Phe Gly Val Lys Gln Ile Ala Thr Gly Val Ser Asp Thr Ile Cys Glu
    130                 135                 140

Pro Cys Pro Val Gly Phe Phe Ser Asn Val Ser Ser Ala Phe Glu Lys
145                 150                 155                 160

Cys His Pro Trp Thr Ser Cys Glu Thr Lys Asp Leu Val Val Gln Gln
                165                 170                 175

Ala Gly Thr Asn Lys Thr Asp Val Val Cys Gly Pro Gln Asp Arg Leu
            180                 185                 190

Arg Ala Leu Val Val Ile Pro Ile Ile Phe Gly Ile Leu Phe Ala Ile
        195                 200                 205

Leu Leu Val Leu Val Phe Ile Lys Lys Val Ala Lys Lys Pro Thr Asn
    210                 215                 220

Lys Ala Pro His Pro Lys Gln Glu Pro Gln Glu Ile Asn Phe Pro Asp
225                 230                 235                 240

Asp Leu Pro Gly Ser Asn Thr Ala Ala Pro Val Gln Glu Thr Leu His
                245                 250                 255

Gly Cys Gln Pro Val Thr Gln Glu Asp Gly Lys Glu Ser Arg Ile Ser
            260                 265                 270

Val Gln Glu Arg Gln
        275


<210>  4
<211>  310
<212>  PRT
<213>  Homo sapiens

<400>  4

Met Arg Arg Ala Ala Leu Trp Leu Trp Leu Cys Ala Leu Ala Leu Ser
1               5                   10                  15

Leu Gln Pro Ala Leu Pro Gln Ile Val Ala Thr Asn Leu Pro Pro Glu
            20                  25                  30

Asp Gln Asp Gly Ser Gly Asp Asp Ser Asp Asn Phe Ser Gly Ser Gly
        35                  40                  45

Ala Gly Ala Leu Gln Asp Ile Thr Leu Ser Gln Gln Thr Pro Ser Thr
    50                  55                  60

Trp Lys Asp Thr Gln Leu Leu Thr Ala Ile Pro Thr Ser Pro Glu Pro
65                  70                  75                  80

Thr Gly Leu Glu Ala Thr Ala Ala Ser Thr Ser Thr Leu Pro Ala Gly
                85                  90                  95

Glu Gly Pro Lys Glu Gly Glu Ala Val Val Leu Pro Glu Val Glu Pro
            100                 105                 110

Gly Leu Thr Ala Arg Glu Gln Glu Ala Thr Pro Arg Pro Arg Glu Thr
        115                 120                 125

Thr Gln Leu Pro Thr Thr His Gln Ala Ser Thr Thr Thr Ala Thr Thr
    130                 135                 140

Ala Gln Glu Pro Ala Thr Ser His Pro His Arg Asp Met Gln Pro Gly
145                 150                 155                 160

His His Glu Thr Ser Thr Pro Ala Gly Pro Ser Gln Ala Asp Leu His
                165                 170                 175

Thr Pro His Thr Glu Asp Gly Gly Pro Ser Ala Thr Glu Arg Ala Ala
            180                 185                 190

Glu Asp Gly Ala Ser Ser Gln Leu Pro Ala Ala Glu Gly Ser Gly Glu
        195                 200                 205

Gln Asp Phe Thr Phe Glu Thr Ser Gly Glu Asn Thr Ala Val Val Ala
    210                 215                 220

Val Glu Pro Asp Arg Arg Asn Gln Ser Pro Val Asp Gln Gly Ala Thr
225                 230                 235                 240

Gly Ala Ser Gln Gly Leu Leu Asp Arg Lys Glu Val Leu Gly Gly Val
                245                 250                 255

Ile Ala Val Gly Leu Val Gly Leu Ile Phe Ala Val Cys Leu Val Gly
            260                 265                 270

Phe Met Leu Tyr Arg Met Lys Lys Lys Asp Glu Gly Ser Tyr Ser Leu
        275                 280                 285

Glu Glu Pro Lys Gln Ala Asn Gly Gly Ala Tyr Gln Lys Pro Thr Lys
    290                 295                 300

Gln Glu Glu Phe Tyr Ala
305                 310


<210>  5
<211>  7820
<212>  DNA
<213>  Artificial

<220>
<223>  lentiviral vector LeGO iG2

<400>  5
gtcgacggat cgggagatct cccgatcccc tatggtgcac tctcagtaca atctgctctg      60

atgccgcata gttaagccag tatctgctcc ctgcttgtgt gttggaggtc gctgagtagt     120

gcgcgagcaa aatttaagct acaacaaggc aaggcttgac cgacaattgc atgaagaatc     180

tgcttagggt taggcgtttt gcgctgcttc gcgatgtacg ggccagatat acgcgttgac 240

attgattatt gactagttat taatagtaat caattacggg gtcattagtt catagcccat 300

atatggagtt ccgcgttaca taacttacgg taaatggccc gcctggctga ccgcccaacg 360

acccccgccc attgacgtca ataatgacgt atgttcccat agtaacgcca atagggactt 420

tccattgacg tcaatgggtg gagtatttac ggtaaactgc ccacttggca gtacatcaag 480

tgtatcatat gccaagtacg ccccctattg acgtcaatga cggtaaatgg cccgcctggc 540

attatgccca gtacatgacc ttatgggact ttcctacttg gcagtacatc tacgtattag 600

tcatcgctat taccatggtg atgcggtttt ggcagtacat caatgggcgt ggatagcggt 660

ttgactcacg gggatttcca agtctccacc ccattgacgt caatgggagt ttgttttggc 720

accaaaatca acgggacttt ccaaaatgtc gtaacaactc cgccccattg acgcaaatgg 780

gcggtaggcg tgtacggtgg gaggtctata taagcagcgc gttttgcctg tactgggtct 840

ctctggttag accagatctg agcctgggag ctctctggct aactagggaa cccactgctt 900

aagcctcaat aaagcttgcc ttgagtgctt caagtagtgt gtgcccgtct gttgtgtgac 960

tctggtaact agagatccct cagacccttt tagtcagtgt ggaaaatctc tagcagtggc 1020

gcccgaacag ggacttgaaa gcgaaaggga aaccagagga gctctctcga cgcaggactc 1080

ggcttgctga agcgcgcacg gcaagaggcg aggggcggcg actggtgagt acgccaaaaa 1140

ttttgactag cggaggctag aaggagagag atgggtgcga gagcgtcagt attaagcggg 1200

ggagaattag atcgcgatgg gaaaaaattc ggttaaggcc agggggaaag aaaaaatata 1260

aattaaaaca tatagtatgg gcaagcaggg agctagaacg attcgcagtt aatcctggcc 1320

tgttagaaac atcagaaggc tgtagacaaa tactgggaca gctacaacca tcccttcaga 1380

caggatcaga agaacttaga tcattatata atacagtagc aaccctctat tgtgtgcatc 1440

aaaggataga gataaaagac accaaggaag ctttagacaa gatagaggaa gagcaaaaca 1500

aaagtaagac caccgcacag caagcggccg gccgcgctga tcttcagacc tggaggagga 1560

gatatgaggg acaattggag aagtgaatta tataaatata aagtagtaaa aattgaacca 1620

ttaggagtag cacccaccaa ggcaaagaga agagtggtgc agagagaaaa aagagcagtg 1680

ggaataggag ctttgttcct tgggttcttg ggagcagcag gaagcactat gggcgcagcg 1740

tcaatgacgc tgacggtaca ggccagacaa ttattgtctg gtatagtgca gcagcagaac 1800

aatttgctga gggctattga ggcgcaacag catctgttgc aactcacagt ctggggcatc 1860

aagcagctcc aggcaagaat cctggctgtg gaaagatacc taaaggatca acagctcctg 1920

gggatttggg gttgctctgg aaaactcatt tgcaccactg ctgtgccttg gaatgctagt 1980

tggagtaata aatctctgga acagatttgg aatcacacga cctggatgga gtgggacaga 2040

gaaattaaca attacacaag cttaatacac tccttaattg aagaatcgca aaaccagcaa 2100

gaaaagaatg aacaagaatt attggaatta gataaatggg caagtttgtg gaattggttt 2160

aacataacaa attggctgtg gtatataaaa ttattcataa tgatagtagg aggcttggta 2220

ggtttaagaa tagtttttgc tgtactttct atagtgaata gagttaggca gggatattca 2280

ccattatcgt ttcagaccca cctcccaacc ccgaggggac ccgacaggcc cgaaggaata 2340

gaagaagaag gtggagagag agacagagac agatccattc gattagtgaa cggatcggca 2400

ctgcgtgcgc caattctgca gacaaatggc agtattcatc cacaatttta aaagaaaagg 2460

ggggattggg gggtacagtg caggggaaag aatagtagac ataatagcaa cagacataca 2520

aactaaagaa ttacaaaaac aaattacaaa aattcaaaat tttcgggttt attacaggga 2580

cagcagagat ccagtttggt tagtaccggg cccgctctag tcgaggtcga cggtatcgat 2640

aagctcgctt cacgagattc cagcaggtcg agggacctaa taacttcgta tagcatacat 2700

tatacgaagt tatattaagg gttccaagct taagcggccg gccgctgaaa gaccccacct 2760

gtaggtttgg caagctagct gcagtaacgc cattttgcaa ggcatggaaa aataccaaac 2820

caagaataga gaagttcaga tcaagggcgg gtacatgaaa atagctaacg ttgggccaaa 2880

caggatatct gcggtgagca gtttcggccc cggcccgggg ccaagaacag atggtcaccg 2940

cagtttcggc cccggcccga ggccaagaac agatggtccc cagatatggc ccaaccctca 3000

gcagtttctt aagacccatc agatgtttcc aggctccccc aaggacctga aatgaccctg 3060

cgccttattt gaattaacca atcagcctgc ttctcgcttc tgttcgcgcg cttctgcttc 3120

ccgagctcta taaaagagct cacaacccct cactcggcgc gccagtcctc cgattgactg 3180

agtcgcccgg atcccagtgt ggtggtacgg gaattcctgc aggcctcgac gagggccggc 3240

gcgccgcggc cgctacgtaa attccgcccc tctccctccc ccccccctaa cgttactggc 3300

cgaagccgct tggaataagg ccggtgtgcg tttgtctata tgttattttc caccatattg 3360

ccgtcttttg gcaatgtgag ggcccggaaa cctggccctg tcttcttgac gagcattcct 3420

aggggtcttt cccctctcgc caaaggaatg caaggtctgt tgaatgtcgt gaaggaagca 3480

gttcctctgg aagcttcttg aagacaaaca acgtctgtag cgaccctttg caggcagcgg 3540

aaccccccac ctggcgacag gtgcctctgc ggccaaaagc cacgtgtata agatacacct 3600

gcaaaggcgg cacaacccca gtgccacgtt gtgagttgga tagttgtgga aagagtcaaa 3660

tggctctcct caagcgtatt caacaagggg ctgaaggatg cccagaaggt accccattgt 3720

atgggatctg atctggggcc tcggtgcaca tgctttacat gtgtttagtc gaggttaaaa 3780

aaacgtctag gccccccgaa ccacggggac gtggttttcc tttgaaaaac acgatgataa 3840

tatggccaca accatggtga gcaagggcga ggagctgttc accggggtgg tgcccatcct 3900

ggtcgagctg gacggcgacg taaacggcca caagttcagc gtgtccggcg agggcgaggg 3960

cgatgccacc tacggcaagc tgaccctgaa gttcatctgc accaccggca agctgcccgt 4020

gccctggccc accctcgtga ccaccctgac ctacggcgtg cagtgcttca gccgctaccc 4080

cgaccacatg aagcagcacg acttcttcaa gtccgccatg cccgaaggct acgtccagga 4140

gcgcaccatc ttcttcaagg acgacggcaa ctacaagacc cgcgccgagg tgaagttcga 4200

gggcgacacc ctggtgaacc gcatcgagct gaagggcatc gacttcaagg aggacggcaa 4260

catcctgggg cacaagctgg agtacaacta caacagccac aacgtctata tcatggccga 4320

caagcagaag aacggcatca aggtgaactt caagatccgc cacaacatcg aggacggcag 4380

cgtgcagctc gccgaccact accagcagaa cacccccatc ggcgacggcc ccgtgctgct 4440

gcccgacaac cactacctga gcacccagtc cgccctgagc aaagacccca acgagaagcg 4500

cgatcacatg gtcctgctgg agttcgtgac cgccgccggg atcactctcg gcatggacga 4560

gctgtacaag taaagcggcc ggccgccagc acagtggtcg aaattcgtcg agggacctaa 4620

taacttcgta tagcatacat tatacgaagt tatacatgtt taagggttcc ggttccacta 4680

ggtacaattc gatatcaagc ttatcgataa tcaacctctg gattacaaaa tttgtgaaag 4740

attgactggt attcttaact atgttgctcc ttttacgcta tgtggatacg ctgctttaat 4800

gcctttgtat catgctattg cttcccgtat ggctttcatt ttctcctcct tgtataaatc 4860

ctggttgctg tctctttatg aggagttgtg gcccgttgtc aggcaacgtg gcgtggtgtg 4920

cactgtgttt gctgacgcaa cccccactgg ttggggcatt gccaccacct gtcagctcct 4980

ttccgggact ttcgctttcc ccctccctat tgccacggcg gaactcatcg ccgcctgcct 5040

tgcccgctgc tggacagggg ctcggctgtt gggcactgac aattccgtgg tgttgtcggg 5100

gaaatcatcg tcctttcctt ggctgctcgc ctgtgttgcc acctggattc tgcgcgggac 5160

gtccttctgc tacgtccctt cggccctcaa tccagcggac cttccttccc gcggcctgct 5220

gccggctctg cggcctcttc cgcgtcttcg ccttcgccct cagacgagtc ggatctccct 5280

ttgggccgcc tccccgcatc gataccgtcg acctcgatcg agacctagaa aaacatggag 5340

caatcacaag tagcaataca gcagctacca atgctgattg tgcctggcta gaagcacaag 5400

aggaggagga ggtgggtttt ccagtcacac ctcaggtacc tttaagacca atgacttaca 5460

aggcagctgt agatcttagc cactttttaa aagaaaaggg gggactggaa gggctaattc 5520

actcccaacg aagacaagat atccttgatc tgtggatcta ccacacacaa ggctacttcc 5580

ctgattggca gaactacaca ccagggccag ggatcagata tccactgacc tttggatggt 5640

gctacaagct agtaccagtt gagcaagaga aggtagaaga agccaatgaa ggagagaaca 5700

cccgcttgtt acaccctgtg agcctgcatg ggatggatga cccggagaga gaagtattag 5760

agtggaggtt tgacagccgc ctagcatttc atcacatggc ccgagagctg catccggact 5820

gtactgggtc tctctggtta gaccagatct gagcctggga gctctctggc taactaggga 5880

acccactgct taagcctcaa taaagcttgc cttgagtgct tcaagtagtg tgtgcccgtc 5940

tgttgtgtga ctctggtaac tagagatccc tcagaccctt ttagtcagtg tggaaaatct 6000

ctagcagcat gtgagcaaaa ggccagcaaa aggccaggaa ccgtaaaaag gccgcgttgc 6060

tggcgttttt ccataggctc cgcccccctg acgagcatca caaaaatcga cgctcaagtc 6120

agaggtggcg aaacccgaca ggactataaa gataccaggc gtttccccct ggaagctccc 6180

tcgtgcgctc tcctgttccg accctgccgc ttaccggata cctgtccgcc tttctccctt 6240

cgggaagcgt ggcgctttct catagctcac gctgtaggta tctcagttcg gtgtaggtcg 6300

ttcgctccaa gctgggctgt gtgcacgaac cccccgttca gcccgaccgc tgcgccttat 6360

ccggtaacta tcgtcttgag tccaacccgg taagacacga cttatcgcca ctggcagcag 6420

ccactggtaa caggattagc agagcgaggt atgtaggcgg tgctacagag ttcttgaagt 6480

ggtggcctaa ctacggctac actagaagaa cagtatttgg tatctgcgct ctgctgaagc 6540

cagttacctt cggaaaaaga gttggtagct cttgatccgg caaacaaacc accgctggta 6600

gcggtggttt ttttgtttgc aagcagcaga ttacgcgcag aaaaaaagga tctcaagaag 6660

atcctttgat cttttctacg gggtctgacg ctcagtggaa cgaaaactca cgttaaggga 6720

ttttggtcat gagattatca aaaaggatct tcacctagat ccttttaaat taaaaatgaa 6780

gttttaaatc aatctaaagt atatatgagt aaacttggtc tgacagttac caatgcttaa 6840

tcagtgaggc acctatctca gcgatctgtc tatttcgttc atccatagtt gcctgactcc 6900

ccgtcgtgta gataactacg atacgggagg gcttaccatc tggccccagt gctgcaatga 6960

taccgcgaga cccacgctca ccggctccag atttatcagc aataaaccag ccagccggaa 7020

gggccgagcg cagaagtggt cctgcaactt tatccgcctc catccagtct attaattgtt 7080

gccgggaagc tagagtaagt agttcgccag ttaatagttt gcgcaacgtt gttgccattg 7140

ctacaggcat cgtggtgtca cgctcgtcgt ttggtatggc ttcattcagc tccggttccc 7200

aacgatcaag gcgagttaca tgatccccca tgttgtgcaa aaaagcggtt agctccttcg 7260

gtcctccgat cgttgtcaga agtaagttgg ccgcagtgtt atcactcatg gttatggcag 7320

cactgcataa ttctcttact gtcatgccat ccgtaagatg cttttctgtg actggtgagt 7380

actcaaccaa gtcattctga gaatagtgta tgcggcgacc gagttgctct tgcccggcgt 7440

caatacggga taataccgcg ccacatagca gaactttaaa agtgctcatc attggaaaac 7500

gttcttcggg gcgaaaactc tcaaggatct taccgctgtt gagatccagt tcgatgtaac 7560

ccactcgtgc acccaactga tcttcagcat cttttacttt caccagcgtt tctgggtgag 7620

caaaaacagg aaggcaaaat gccgcaaaaa agggaataag ggcgacacgg aaatgttgaa 7680

tactcatact cttccttttt caatattatt gaagcattta tcagggttat tgtctcatga 7740

gcggatacat atttgaatgt atttagaaaa ataaacaaat aggggttccg cgcacatttc 7800

cccgaaaagt gccacctgac 7820

<210> 6
<211> 28
<212> DNA
<213> Artificial

<220>
<223> oligonucleotide primer

<400> 6
atagcggccg ccaccatgcc acctcctc 28

<210> 7
<211> 27
<212> DNA
<213> Artificial

<220>
<223> oligonucleotide primer

<400> 7
tattgtacat cacctggtgc tccaggt 27

<210> 8
<211> 35
<212> DNA
<213> Artificial

<220>
<223> oligonucleotide primer

<400> 8
atagcggccg ccaccatggc caactgcgag ttcag 35


<210> 9
<211> 36
<212> DNA
<213> Artificial

<220>
<223> oligonucleotide primer

<400> 9
tattgtacat cagatctcag atgtgcaaga tgaatc 36


<210> 10
<211> 34
<212> DNA
<213> Artificial

<220>
<223> oligonucleotide primer

<400> 10
atagcggccg ccaccatggt tcgtctgcct ctgc 34


<210> 11
<211> 29
<212> DNA
<213> Artificial

<220>
<223> oligonucleotide primer

<400> 11
tattgtacat cactgtctct cctgcactg 29


<210> 12
<211> 28
<212> DNA
<213> Artificial

<220>
<223> oligonucleotide primer

<400>  12
atagcggccg ccaccatgag gcgcgcgg                                          28


<210>  13
<211>  34
<212>  DNA
<213>  Artificial

<220>
<223>  oligonucleotide primer

<400>  13
tattgtacat caggcataga attcctcctg tttg                                   34